# EUROPEAN PATENT SPECIFICATION

(11) **EP 0 512 017 B1**
(45) Date of publication and mention of the grant of the patent: **18.06.1997**
(21) Application number: 91903143.5
(22) Date of filing: 25.01.1991
(51) Int. Cl.: C12N 15/86, C12N 15/35, C12N 15/45, C12N 15/47, C12N 15/48, C12N 15/50, A61K 39/295

(54) **VACCINES**
VAKZINE
VACCINS

(30) Priority: 25.01.1990 GB 9001766
(43) Date of publication of application: 11.11.1992
(73) Proprietor: THE UNIVERSITY COURT OF THE UNIVERSITY OF GLASGOW, Glasgow G12 8QQ (GB)
(72) Inventor: SPIBEY, Norman, Glasgow G61 2LZ (GB)
(74) Representative: Horner, Martin Grenville
(86) International application number: GB9100107
(87) International publication number: WO9111525

(56) References cited:
- EP-A- 0 181 117
- Virus Research, volume 14, 1989, Elsevier Science Publishers B.V.; N. Spibey et al.: "Identification and nucleotide sequence of the early region 1 from canine adenovirus types 1 and 2" pages 241-256
- Gene, volume 55, 1987, Elsevier Science B.V.; M. Shinagawa et al.: "Phylogenetic relationships between adenoviruses as inferred from nucleotide sequences of inverted terminal repeats", page 85
- Journal of Virology, volume 54, no. 3, June 1985, American Society for Microbiology; I. Saito et al.: "Construction of nondefective adenovirus type 5 bearing a 2.8-kilobase hepatitis B virus DNA near the right end of its genome", pages 711-719
- J. Gen. Virol., Volume 70, 1989, SGM; N. Spibey et al.: "Molecular cloning and restriction endonuclease mapping oftwo strains of canine adenovirus type 2", pages 165-172
- Technological Advances in Vaccine Development, 1988; Alan R. Liss, Inc., F.L. Graham et al.: "Cloning and expression of glycoprotein genes in human adenovirus vectors 1", pages 243-253
- Trends in Biotechnology, volume 8, no. 4, April 1990, Elsevier Science Publishers Ltd, (Cambridge, GB); F.L. Graham: "Adenoviruses as expression vectors and recombinant vaccines", pages 85-87

## Description

The present invention relates to vaccines for use in carnivora and in particular to viral vaccines against diseases such as rabies, canine and feline parvovirus, and feline leukaemia virus. These would have distinct advantages over those in current use.

Recently there has been proposed a DNA vector system wherein a gene coding for the target antigen is inserted into a live adenovirus which is then formulated in an enteric coated dosage form.

However, many adenoviruses are known. The selection of a suitable virus to act as a vector for the gene, and the identification of a suitable non-essential region as a site for insertion of the gene pose a challenge. In particular, the insertion site must be non-essential for the viable replication of the virus and its effective operation in vivo. Moreover, the insertion site must be capable of accepting a considerable amount of new genetic material, whilst ensuring that the virus continues to replicate. The CAV-2 adenovirus is known to be a safe vector but its DNA comprises some 31,000 base pairs which must be searched to identify a suitable site or indeed to establish whether a suitable site exists or not.

The present inventors have now identified a suitable non-essential region in strains of live non-pathogenic immunogenic canine CAV-2 type adenovirus and have succeeded in inserting genes from pathogenic carnivora viruses with suitable expression control systems without prejudicing the stable reproducibility of the adenovirus vector.

A recombinant adenovirus for producing antibodies or cell mediated immunity to an infectious organism in carnivora, which comprises a live non-pathogenic immunogenic viable canine adenovirus modified so as to contain a gene coding for the antigen which corresponds to said antibodies or induces said cell mediated immunity, in association with an effective promoter for said gene formed and arranged for expression of said antigen or cell mediated immunity elements in immunogenic non-pathogenic quantities; the promoter-gene sequence being introduced into a region of the viral genome which extends from the SmaI site close to the end of the inverted terminal repeat (ITR) up to the promoter for the early region 4 (E4).

Preferably said canine adenovirus is a CAV-2 vaccine type strain of known long term safety modified so as to contain the inserted gene at a position close to the right hand terminus of the viral genome.

The inverted terminal repeat (ITR) is a DNA sequence found at both ends of the adenoviral genome. All adenoviruses examined to date have been shown to contain an ITR; however the lengths of these vary between serotypes. The ITR in CAV-2 is 197 base pairs (i.e. the region 0 to 197). The ITRs contain sequences which are essential for viral DNA replication and efficient packaging of the viral genomic DNA. However, we have identified a region at the end of the ITR of CAV-2 which can accommodate a substantial amount of extra DNA. This region extends from approximately the position of the Sma I site close to the end of the ITR (and just inside the ITR) up to the promoter for early region 4 (E4). Thus the Sma I site provides a convenient position into which foreign genes may be inserted. Other restriction sites may also be engineered into this region.

The SmaI site is a very convenient site at which to insert the gene, notwithstanding that it lies just inside the ITR. However, the insertion of material further into the ITR-is unlikely to be successful as viral functions require the ITR's at the opposite ends of the virus to be able to line up together, which requires close sequence homology between the two ITR's.

The chosen non-pathogenic CAV-2 viral genome has been cloned into bacterial plasmids as a series of overlapping restriction fragments. A cloned fragment was required which spanned the Sma l site mentioned above and also included the righthand terminus of the virus. A clone carrying the 3.0 kb Sal l B fragment was therefore chosen.

Various genes may be inserted in the adenovirus DNA in accordance with the present invention to provide protection against a wide range of diseases and many such genes are already known in the art - the problem heretofore having been to provide a safe, convenient and effective vector for the genes.

Genes which may usefully be inserted include:
1) Gene(s) for the capsid proteins of canine parvovirus. There is in reality only one gene. However, differential splicing results in the production of two viral capsid proteins.
2) The genes for the capsid proteins of feline panleukopenia, which is a parvovirus very closely related to the canine parvovirus.
3) Genes (or segments) coding for the peplomeres of canine and feline coronavirus.
4) Genes for the hemagglutanin and capsid antigens of canine distemper virus.
5) The gene for the envelope glycoprotein of feline leukemia virus.
6) The gene for the envelope glycoprotein of rabies virus (various strains)
7) The gene for the envelope glycoprotein of feline immunodeficiency virus (FIV).

It is also possible that only fragments of genes may be used (where these are sufficient to generate an immunogenic protein or immunogenic cell response) rather than the complete sequence as found in the wild organism. Where available, synthetic genes may also be used. However, the present invention can be used with a wide variety of genes and is not limited to those set out above.

In some cases the gene for a particular antigen may contain a large number of introns or may be from an RNA virus, in these cases a DNA copy (cDNA) may be used.

In order for successful expression of the gene to occur, it must be inserted together with a suitable promoter sequence. Suitable promoter sequences for the genes mentioned above are known. The promoter is selected to give optimal expression of immunogenic protein or optimal cell mediated immunity according to known criteria.

The protein produced by expression in vivo in a recombinant virus-infected cell may be itself immunogenic. Moreover, the gene may produce more than one effective protein; or more than one foreign gene may be inserted in the viral genome.

Thus with the recombinant virus of the present invention, it is possible to provide protection in carnivora against a wide variety of diseases.

In a second aspect the present invention provides a method of preparing a recombinant adenovirus for producing antibodies or cell mediated immunity to an infectious organism in carnivora, which comprises modifying a live non-pathogenic immunogenic viable canine adenovirus so as to contain a gene coding for the antigen which corresponds to said antibodies or induces said cell mediated immunity, in association with an effective promoter for said gene formed and arranged for expression of said antigen or cell mediated immunity elements in immunogenic non-pathogenic quantities; the promoter-gene sequence being introduced into a region of the viral genome which extends from the SmaI site close to the end of the inverted terminal repeat (ITR) up to the promoter for the early region 4 (E4).

Generally, the promoter-gene construct is cloned into a DNA sequence which is only a part of the entire viral genome. This sequence may be present in a plasmid which allows successful cloning to produce many copies of the sequence. The cloned sequence may then be included in the complete viral genome by the use of restriction enzymes corresponding to the restriction sites at each end of the sequence. A third aspect of the invention relates to a plasmid containing such a partial viral DNA sequence containing a complete promoter-gene construct.

The DNA sequences of the virus and the construct may be identical to the native form or may be equivalent thereto in terms of the protein expressed. Moreover, the immunological protein (and hence the DNA) may have additions, deletions or substitutions immaterial to its immunological effect.

The recombinant virus may be conveniently replicated by transfection into a cell line expressing canine adenovirus E1a proteins, which switch on the viral gene. Accordingly the invention also provides an admixture of the recombinant virus and E1a proteins.

A fourth aspect of the invention provides a vaccine formulation which comprises tne recombinant adenovirus in association with an acceptable carrier. The vaccine may be formulated for administration by oral dosage (e.g. as an enteric coated tablet), by injection or otherwise.

Embodiments of the invention will now be described by way of example only. Standard methods are as described in "Molecular Cloning - A Laboratory Manual", Second Edition, Sambrook, J., Fritsch, E.F., and Maniatis, T., Cold Spring Harbor Laboratory Press 1989.

### Example 1 (in vitro ligation)

A gene of interest such as the rabies glycoprotein gene is first put under the control of a chosen promoter, (a number of different promoters are possible). The promoter-gene construct is then cloned into the Sma l site of the Sal l B fragment contained within a plasmid. This results in a plasmid which carries the right terminal 3.0kb of CAV-2 with a chosen piece of DNA inserted just inside the boundary of the ITR.

Briefly, the modified right terminal section of the adenovirus genome is then incorporated into a complete viral genome as follows: Viral DNA is purified and cut with the restriction enzyme Sal l, the cloned terminal Sal l fragment containing the promoter-gene construction is then released from the carrier plasmid and mixed with the cut viral DNA. The DNA fragments are then ligated back together.

A number of steps are taken to minimise the reformation of wild type genomes and to maximise the formation of the "correct" recombinant. For example the Sal I digested viral DNA can be run through a sucrose gradient in order to remove the right hand terminal Sal I fragment prior to ligation with the recombinant right hand terminal fragment.

The recombinant viral DNA is reintroduced to cultured dog cells using standard procedures in order to culture the virus. Individual viral plaques are picked and expanded in order that their genomic DNAs can be examined to confirm the presence of the recombinant gene.

Ligation products have been made by this method which incorporate the rabies gene, and the feline leukemia gene.

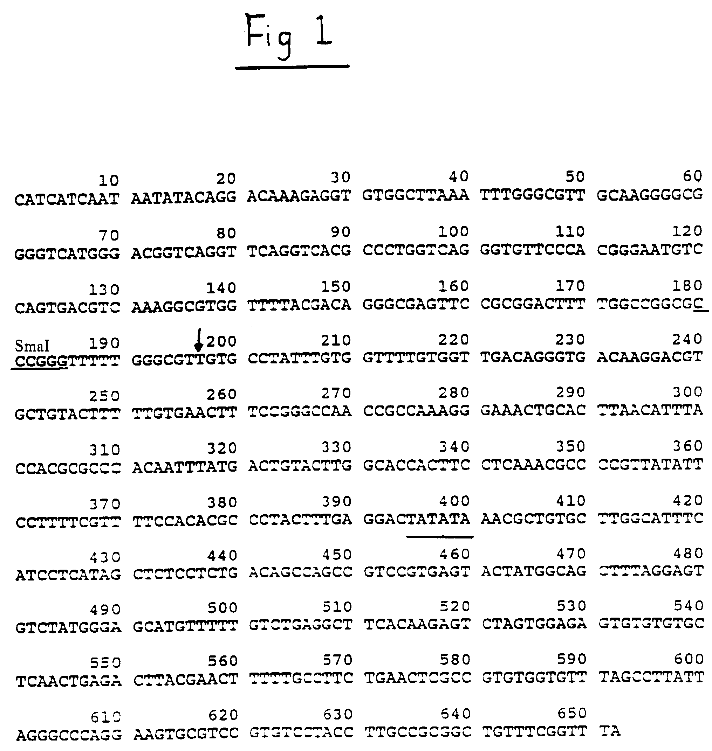

The DNA sequence of the right hand terminal region of the canine adenovirus type 2 genome is presented in Figure 1. The sequence is shown in the conventional 5' to 3' direction numbering from the righthand terminus. For clarity only the upper strand is shown. Various features are indicated: (1) the end of the inverted terminal repeat (ITR) at position 197; (2) the Sma I restriction enzyme cleavage site at position 180-185; (3) the TATA box of the E4 transcription unit at position 395-400.

### Example 2 (homologous recombination)

In Example 1 the target genes have been cloned into the SmaI site of the SalI B clone and subsequently the recombinant viruses were generated by in vitro ligation of viral DNA and plasmid fragments. In a variation of this method we have taken the recombinant SalI B clones (i.e. with the target genes inserted in the usual place) and extended them past the SalI site up to the third Kpn I from the right hand terminus (see Fig.3 of N. Spibey and H.M.A. Cavanagh, J.Gen. Virol (1989), 70, 165-172). This was achieved by simply ligating the KpnI B fragment (which had been previously cloned into a standard bacterial cloning vector) into the appropriately cut recombinant SalI B plasmids. Because these recombinant right-hand terminal clones extend beyond the SalI site their incorporation into intact viral DNA was carried out via homologous recombination rather than by in vitro ligation.

The generation of recombinant viruses by homologous recombination is well established, briefly our methods are as follows: Viral DNA-protein complex was prepared from purified virions using standard protocols. A sample of the DNA protein complex was digested with the restriction enzyme SalI. A recombinant plasmid comprising the right terminal portion of the CAV-2 genome, extending from approximately 74 map units (the Kpn l site) up to the terminus (100 map units), with the chosen promoter-target gene construction inserted into the SmaI site near the ITR boundary, was taken and digested with a restriction enzyme such that plasmid was linearised but not cut within the CAV or target gene sequences. The two DNA samples (viral DNA protein complex and linearised plasmid) were then mixed in a molar ratio of 1:20 and transfected into a recipient cell line whose production is described hereafter.

### Example 3 (Production of Ela Protein - Expressing Recipient Cell Line).

Primary dog kidney cell cultures and established dog kidney cell cultures (MDCK cells) were taken and transfected with a mixture of two plasmids, (1) pGRIC, which comprises the left terminal EcoRI C fragment of CAV-2 cloned into the routine cloning vector bluescript. (2) pSV2-Neo, this plasmid contains the neomycin resistance gene under control of the Sv40 virus early promoter. The purpose of these transfections was to produce cell lines which constitutively produce the canine adenovirus Ela proteins (these proteins are encoded within the EcoRI C fragment, Spibey et al., Virus Research, 14, (1989) 241). We hypothesised that such a cell line would be more efficient at replicating transfected viral DNA because the El proteins are already present. The El proteins have a number of functions, however their major role can be regarded as regulatory i.e. they switch on other viral genes. Therefore if these proteins are already present then the transfected viral DNA may have a greater chance of being replicated and therefore avoid the cellular degradative processes. The plasmid conferring neomycin resistance was used as a co-selectable marker, i.e. individual clones of cells were selected on the basis of their neomycin resistance and then subsequently analysed for their production of CAV-2 Ela proteins. Transformed (i.e. Ela expressing) cell lines were obtained from both primary and established dog kidney cultures.

### Example 4 (Transfection Method)

All transfections were carried out as follows.
Day 1 Trypsinize cells and dilute them to a concentration of 1-1.5 x 10⁵ cells per ml. The cells are then plated at 10⁶ cells per 100mm dish (or the equivalent number per 175cm² flask).
Day 2 Wash cells once in PBS. To each dish add 5ml of serum free media containing 20ug of DNA to be transfected and 50ul of DEAE dextran (5mg/ml stock). Ensure DNA/dextran solution is well mixed before adding to the cells.

After 4-6 hours remove DNA/dextran solution and add 5ml of serum free media containing 10% DMSO, leave for 1-2 min then remove and add complete media containing 0.1mM chloroquine diphosphate. After 4 hours replace media with fresh complete media.

Incubate cells for required period of time. This can be up to 10 days to allow for a viral infection to spread throughout the whole culture.

Neomycin resistant clones were selected using standard protocols. Briefly, after transfection cells were allowed 24 hours in normal complete media before the addition of neomycin (800 ug/ml final concentration). The neomycin selection was continued for 7-10 days with media changed every 2 days. Resistant clones were identified, picked and expanded in neomycin free media for 3-4 days. A further round of dilution cloning in neomycin containing media was then carried out.

All cellular incubations were carried out at 37°C.

## Claims

1. A recombinant adenovirus for producing antibodies or cell mediated immunity to an infectious organism in carnivora, which comprises a live non-pathogenic immunogenic viable canine adenovirus modified so as to contain a gene coding for the antigen which corresponds to said antibodies or induces said cell mediated immunity, in association with an effective promoter for said gene formed and arranged for expression of said antigen or cell mediated immunity elements in immunogenic non-pathogenic quantities; the promoter-gene sequence being introduced into a region of the viral genome which extends from the SmaI site close to the end of the inverted terminal repeat (ITR) up to the promoter for the early region 4 (E4).

2. An adenovirus according to claim 1 which is a CAV-2 strain modified to contain said promoter-gene sequence.

3. An adenovirus according to claim 1 or 2 wherein the promoter-gene sequence is introduced into the SmaI site.

4. An adenovirus according to any preceding claim wherein the introduced gene is a gene or immunogenic gene fragment selected from:
(a) gene(s) for the capsid proteins of canine parvovirus;
(b) genes for the capsid proteins of feline panleukopenia;
(c) genes for the peplomeres of canine and feline coronavirus;
(d) genes for the hemagglutanin and capsid antigens of canine distemper virus;
(e) genes for the envelope glycoprotein of feline leukaemia virus;
(f) gene for the envelope glycoprotein of rabies virus; and
(g) gene for the envelope glycoprotein of feline immunodeficiency virus.

5. A recombinant adenovirus according to any preceding claim admixed with canine adenovirus Ela proteins.

6. A method of preparing a recombinant adenovirus for producing antibodies or cell mediated immunity to an infectious organism in carnivora, which comprises modifying a live non-pathogenic immunogenic viable canine adenovirus so as to contain a gene coding for the antigen which corresponds to said antibodies or induces said cell mediated immunity, in association with an effective promoter for said gene formed and arranged for expression of said antigen or cell mediated immunity elements in immunogenic non-pathogenic quantities; the promoter-gene sequence being introduced into a region of the viral genome which extends from the SmaI site close to the end of the inverted terminal repeat (ITR) up to the promoter for the early region 4 (E4).

7. A method according to claim 6 wherein the recombinant virus is replicated by transfection into a cell line expressing canine adenovirus Ela proteins.

8. A plasmid which contains a part of the DNA sequence from said live non-pathogenic immunogenic viable canine adenovirus and a complete promoter-gene sequence according to any of claims 1 to 5.

9. A vaccine formulation which comprises the recombinant adenovirus according to any of claims 1 to 5 together with an acceptable carrier therefor.

## Patentansprüche

1. Rekombinantes Adenovirus zur Herstellung von Antikörpern oder zellvermittelter Immunität gegen einen infektiösen Organismus in Fleischfressern, mit einem lebenden nicht pathogenen immunogenen lebens fähigen Hundeadenovirus, welches so modifiziert ist, daß es ein Gen enthält, welches für das Antigen kodiert, welches zu den Antikörpern gehört oder die zellvermittelte Immunität induziert, in Verbindung mit einem wirksamen Promotor für das Gen, welcher zur Expression des Antigens oder der Elemente zellvermittelter Immunität in immunogenen nicht pathogenen Mengen gebildet und angeordnet ist; wobei die Promotorgensequenz in einen Bereich des viralen Genoms eingeführt ist, welcher sich von der SmaI-Stelle in der Nähe des Endes der umgekehrten terminalen Wiederholung (ITR) bis zum Promotor für den frühen Bereich 4 (E4) erstreckt.

2. Adenovirus gemäß Anspruch 1, wobei es sich um einen CAV-2-Stamm handelt, welcher so modifiziert ist, daß er die Promotorgensequenz enthält.

3. Adenovirus gemäß Anspruch 1 oder 2, bei dem die Promotorgensequenz in die SmaI-Stelle eingefügt ist.

4. Adenovirus gemäß einem der vorhergehenden Ansprüche, bei dem es sich bei dem eingefügten Gen um ein Gen oder immunogenes Genfragment handelt, ausgewählt aus:
(a) einem Gen oder Genen für die Capsidproteine eines Hundeparvovirus;
(b) Genen für die Capsidproteine von Katzenfieber;
(c) Genen für die Peplomere eines Hunde- und Katzencoronavirus;
(d) Genen für die Hämagglutanin- und Capsidantigene eines Hundestaupevirus;
(e) Genen für das Hüllglycoprotein eines Katzenleukämievirus;
(f) einem Gen für das Hüllglycoprotein eines Tollwutvirus; und
(g) einem Gen für das Hüllglycoprotein eines Katzenimmunschwächevirus.

5. Rekombinantes Adenovirus gemäß einem der vorhergehenden Ansprüche, das mit Hundeadenovirus-E1a-Proteinen vermischt ist.

6. Verfahren zur Herstellung eines rekombinanten Adenovirus zur Herstellung von Antikörpern oder zellvermittelter Immunität gegen einen infektiösen Organismus in Fleischfressern, mit der Modifizierung eines lebenden nicht pathogenen immunogenen lebensfähigen Hundeadenovirus, um ein Gen zu enthalten, welches für das Antigen kodiert, welches zu den Antikörpern gehört oder die zellvermittelte Immunität induziert, in Verbindung mit einem wirksamen Promotor für das Gen, welcher zur Expression des Antigens oder der Elemente zellvermittelter Immunität in immunogenen nicht pathogenen Mengen gebildet und angeordnet wird; wobei die Promotorgensequenz in einen Bereich des viralen Genoms eingefügt wird, welcher sich von der SmaI-Stelle in der Nähe des Endes der umgekehrten terminalen Wiederholung (ITR) bis zum Promotor für den frühen Bereich 4 (E4) erstreckt.

7. Verfahren gemäß Anspruch 6, bei dem das rekombinante Virus durch Transfektion in eine Zellinie repliziert wird, welche Hundeadenovirus-E1a-Proteine exprimiert.

8. Plasmid, enthaltend einen Teil der DNS-Sequenz von dem lebenden nicht pathogenen immunogenen lebensfähigen Hundeadenovirus und eine vollständige Promotorgensequenz gemäß einem der Ansprüche 1 bis 5.

9. Impfstoff, enthaltend den rekombinanten Adenovirus gemäß einem der Ansprüche 1 bis 5 zusammen mit einem akzeptablen Träger hierfür.

## Revendications

1. Adénovirus recombinant pour la production d'anticorps ou d'immunité à médiation cellulaire contre un organisme infectieux chez des carnassiers, qui comprend un adénovirus canin viable immunogène non pathogène vivant modifié de façon à contenir un gène codant pour l'antigène qui correspond à ces anticorps ou induit cette immunité à médiation cellulaire, en association avec un promoteur efficace pour ce gène formé et arrangé pour l'expression de cet antigène ou de ces éléments d'immunité à médiation cellulaire en des quantités non pathogènes immunogènes; la séquence promoteur-gène étant introduite dans une région du génome viral qui s'étend du site SmaI proche de l'extrémité de l'unité répétée terminale inverse (ITR) jusqu'au promoteur pour la région précoce 4 (E4).

2. Adénovirus suivant la revendication 1, qui est une souche CAV-2 modifiée pour contenir cette séquence promoteur-gène.

3. Adénovirus suivant les revendications 1 ou 2, dans lequel la séquence promoteur-gène est introduite dans le site SmaI.

4. Adénovirus suivant l'une quelconque des revendications précédentes, dans lequel le gène introduit est un gène ou un fragment de gène immunogène choisi parmi :
(a) un ou des gènes pour les protéines capsidiques de parvovirus canin;
(b) des gènes pour les protéines capsidiques de panleucopénie féline;
(c) des gènes pour les peplomères de coronavirus canin et félin;
(d) des gènes pour l'hémagglutanine et les antigènes capsidiques de virus canin de la maladie des chiens;
(e) des gènes pour la glycoprotéine d'enveloppe du virus de leucémie féline;
(f) un gène pour la glycoprotéine d'enveloppe du virus de la rage; et
(g) un gène pour la glycoprotéine d'enveloppe du virus de l'immunodéficience féline.

5. Adénovirus recombinant suivant l'une quelconque des revendications précédentes mélangé à des protéines E1a d'adénovirus canin.

6. Procédé pour la préparation d'un adénovirus recombinant pour la production d'anticorps ou d'immunité à médiation cellulaire contre un organisme infectieux chez des carnassiers, qui comprend la modification d'un adénovirus canin viable immunogène non pathogène vivant de façon à contenir un gène codant pour l'antigène qui correspond à ces anticorps ou induit cette immunité à médiation cellulaire, en association avec un promoteur efficace pour ce gène formé et arrangé pour l'expression de cet antigène ou de ces éléments d'immunité à médiation cellulaire en des quantités non pathogènes immunogènes; la séquence promoteur-gène étant introduite dans une région du génome viral qui s'étend du site SmaI proche de l'extrémité de l'unité répétée terminale inverse (ITR) jusqu'au promoteur pour la région précoce 4 (E4).

7. Procédé suivant la revendication 6, dans lequel le virus recombinant est répliqué par transfection dans une lignée cellulaire exprimant des protéines E1a d'adénovirus canin.

8. Plasmide qui contient une partie de la séquence d'ADN provenant de cet adénovirus canin viable immunogène non pathogène vivant et une séquence promoteur-gène complète suivant l'une quelconque des revendications 1 à 5.

9. Formulation de vaccin, qui comprend l'adénovirus recombinant suivant l'une quelconque des revendications 1 à 5 avec un support acceptable pour celui-ci.
